# EUROPEAN PATENT APPLICATION

(11) **EP 2 966 062 A1**
(43) Date of publication of application: **13.01.2016**
(21) Application number: 14176491.0
(22) Date of filing: 10.07.2014
(51) Int. Cl.: C07D 215/56, A61K 31/47, A61P 29/00, A61P 25/00

(54) **4-oxo-1,4-dihydroquinoline-3-carboxamide as selective ligand for cannabinoid receptor 2 for diagnosis and therapy**

(71) Applicant: ETH Zurich, 8092 Zurich (CH); Universität Zürich, 8006 Zürich (CH)
(72) Inventor: Slavik, Roger, 8400 Winterthur (CH); Mu, Linjing, 5600 Lenzburg (CH); Ametamey, Simon M., 8045 Zurich (CH)
(74) Representative: Kasche, André

(57) **Abstract**

The present invention is directed to new compounds selectively binding the cannabinoid 2 receptor. In addition, the invention relates to the use of said compounds for determining cannabinoid receptor 2 (CB2)-selective receptor localization and density, preferably in the central nervous system (CNS), the peripheral nervous system (PNS), heart, liver, gastrointestinal tract, spleen, pancreas, kidney, testis, ovary and/or the prostate. Moreover, the invention pertains to the use of said compounds in the diagnosis, prophylaxis and/or therapy of CB2 receptor-related diseases.

## Description

The present invention is directed to new compounds selectively binding the cannabinoid 2 receptor. In addition, the invention relates to the use of said compounds for determining cannabinoid receptor 2 (CB2)-selective receptor localization and density, preferably in the central nervous system (CNS), the peripheral nervous system (PNS), heart, liver, gastrointestinal tract, spleen, pancreas, kidney, testis, ovary and/or the prostate. Moreover, the invention pertains to the use of said compounds in the diagnosis, prophylaxis and/or therapy of CB2 receptor-related diseases.

### Background of the invention

The endocannabinoid system (ECS) comprises the rhodopsin-like G-coupled cannabinoid 1 and 2 receptors (CB1, CB2) which negatively regulate adenylate cyclase, their endogenous lipid ligands or endocannabinoids (ECs) as well as catabolizing and metabolizing enzymes. The ECS has been implicated in a growing number of physiological and pathological functions. Studies in mice deficient in cannabinoid receptors or EC degrading enzymes as well as selective cannabinoid receptor ligands and inhibitors of the EC metabolism have demonstrated the ECS involvement in a variety of physiopathological processes, both in the peripheral and central nervous systems (PNS, CNS) and in various peripheral organs. Such studies indicate that modulation of ECS activity may have therapeutic potential in almost all diseases affecting humans including obesity/metabolic syndrome; diabetes, diabetic complications; neurodegenerative, inflammatory, cardiovascular, liver, gastrointestinal and skin diseases; pain; psychiatric disorders; cachexia; cancer and chemotherapy-induced nausea and vomiting, amongst many others. These investigations have uncovered the remarkable complexity of the ECS. For example, there is an overlap between EC and eicosanoid signaling and there are often opposite effects mediated by cannabinoid 1 and 2 receptors (CB1, CB2) in disease models. The first human trial with peripherally-restricted mixed CB1/2 agonists for pain failed as a result of cardiovascular and metabolic side effects and hepa-totoxicity (Pacher and Kunos, FEBS Journal, 280 (2013) 1918-1943).

CB1 receptors, the most abundant G-protein coupled receptors in the mammalian brain, mediate the socially undesirable psychoactive effects of cannabis. CB1 receptors can also be found in almost all peripheral tissues and cells, albeit at much lower densities.

CB2 receptors are largely restricted to immune and haemopoetic cells, although functionnally relevant expression has been found in specific regions of the brain, the myocardium, gut, endothelial, vascular smooth muscle and Kupffer cells, exocrine and endocrine pancreas, bone and reproductive organs/cells as well as in various tumors.

Dysregulation of the ECS, in most cases up-regulation of the CB1 and/or CB2 receptors and/or increase in tissue levels of EC are associated with various pathologies in mammals, including myocardial infarction, ischemia reperfusion injury, heart failure, cardiomyopathies, atherosclerosis, restenosis, stroke, spinal cord injury, cirrhotic cardiomyopathy, septic shock by live bacteria, hepatic ischaemia reperfusion injury, obesity, non-alcoholic fatty liver disease, diabetic complications, liver fibrosis, cirrhosis, alcohol-induced liver injury, pancreatitis, inflammatory bowel disease, colitis, diverticulitis, nephropathy, neurodegenerative/neuroinflammatory disorders, in particular multiple sclerosis (MS), Alzheimer's disease (AD), Parkinson's disease (PD) and Huntington's disease (HD), spinal cord injury; psychiatric disorders, in particular anxiety and depression schizophrenia, rheumatoid arthritis, cancer (see Pacher and Kunos, FEBS Journal, 280 (2013) 1918-1943, Table 1).

From a diagnostic point of view the identification of ECS dysregulation can assist in identifying ECS-specific diseases.

From a therapeutic standpoint the identification of regional or tissue-specific changes in CB receptor distribution and density is important for CB1 and CB2-selective targeting, which may mitigate unwanted side effects and improve efficacy.

Selective CB2 agonists have been shown to exert beneficial effects in rodent models of myocardial infarction by limiting inflammatory cell infiltration in cardiomyocytes.

The activation of CB1, e.g. by tetrahydrocannabinol (THC), the putative psychoactive ingredient in marijuana, is clearly associated with adverse cardiovascular consequences, a fact that needs to be carefully considered during the preclinical/clinical development of drugs targeting the ECS.

Pasquini et al. (Journal of Medicinal Chemistry, 2011, 54: 5444-5453) discloses the investigation of 4-quinolone-3-carboxamides, in particular of the high affinity as well as highly CB2-selective N-(1-adamantyl)-1-pentyl-8-methoxy-4-oxo-1,4-dihydroquinoline-3-carboxamide, as new potent and selective ligands for the CB2 receptor and their anti-hyperalgesic effects in mice.

Mu et al. (Journal of Neurochemistry, 2013, 126, 616-624) reported on the radiolabelling and *in vitro*/*in vivo* evaluation of N-(1-adamantyl)-1-pentyl-8-methoxy-4-oxo-1,4-dihydroquinoline-3-carboxamide (KD2) as a PET (positron emission tomography) probe radiolabelled with ¹¹C isotope for imaging cannabinoid type 2 receptors. The authors further identify the CB2 receptor as a very promising target for neuroinflammatory and neurodegenerative diseases such as MS, ALS and AD for therapeutic approaches and imaging. The radiolabelled 4-oxoquinoline derivative KD2 demonstrated moderate blood-brain barrier (BBB) passage in an *in vitro* transport assay and exhibited high specific binding towards CB2. In rats a high spleen uptake was shown and a displacement study with a selective CB2 agonist confirmed specificity. Spinal cord slices from ALS patients showed CB2 receptors under disease conditions.

However, the authors concluded from the *in vitro*/*in vivo* characterization of radiolabelled KD2 that this compound has acceptable albeit not ideal properties as a PET tracer, even though affinity and selectivity for CB2 are ideal for CB2 imaging. But the observed relatively high plasma binding of the lipophilic PET tracer proved not to be favorable for brain imaging because plasma binding competes with the BBB passage and can reduce brain uptake of the tracer. The quinolin-4-(1H)-one structure is presumably uncharged at physiological pH, resulting in a relatively high distribution coefficient, i.e. a high logD value at pH 7.4 of 3.29, thus leading to an expected moderate to low brain uptake. Consequently, KD2 distribution in brain was relatively low in *in vivo* PET experiments, i.e. lower than in peripheral tissues in general, and only minute accumulation was observed in all investigated brain regions. The authors suggest KD2 as a lead structure for PET imaging with promising *in vivo* characteristics and good potential for improvement.

It is the objective of the present invention to provide new compounds with high CB2 receptor affinity and selectivity, in particular for use in the diagnosis, prophylaxis and treatment of ECS-associated, in particular CB2 receptor-related diseases, including cardiovascular disease, myocardial infarction, ischemia reperfusion injury, heart failure, cardiomyopathies, atherosclerosis, restenosis, stroke, spinal cord injury, cirrhotic cardiomyopathy, septic shock by live bacteria, hepatic ischaemia reperfusion injury, obesity, non-alcoholic fatty liver disease, diabetes, diabetic complications, obesity/metabolic syndrome; liver, gastrointestinal and skin diseases; liver fibrosis, cirrhosis, alcohol-induced liver injury, pancreatitis, inflammatory bowel disease, colitis, diverticulitis, nephropathy, neurodegenerative/neuroinflammatory disorders, in particular multiple sclerosis (MS), Alzheimer's disease (AD), Parkinson's disease (PD) and Huntington's disease (HD); spinal cord injury, pain; psychiatric disorders, in particular anxiety and depression schizophrenia; rheumatoid arthritis, cachexia, cancer, chemotherapy-induced nausea and vomiting.

In a first aspect the objective of the present invention is solved by the following compounds of formula (I) or (II): 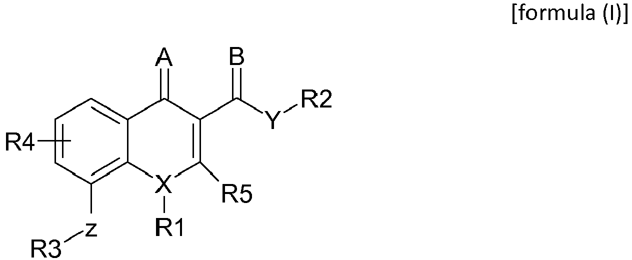 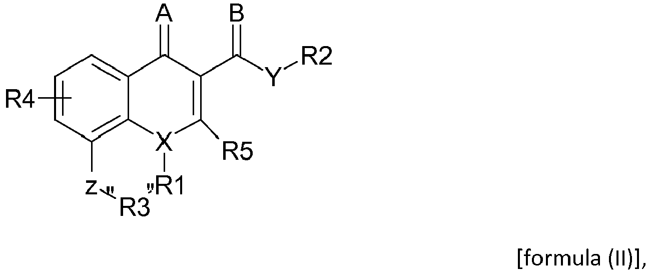 wherein:
A is selected from -O-, -S- and -NR₆-, preferably A is -O-;
B is selected from -O-, -S-, -NR₆-, preferably B is -O-;
X is -N- or -CH-, preferably -N-;
Y is selected from -O-, -NH-, -NR₆-, -S-, substituted or unsubstituted -CH₂- or a direct bond, preferably Y is -NH-;
Z is selected from -O-, -NH-, -S-, substituted or unsubstituted -CH₂- or a direct bond, preferably Z is -O-;
R1 is selected from the group consisting of
   (i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
   (ii) linear or branched, substituted or non-substituted (C₁₋₁₀)alkyl thioether, (C₂₋₁₀)alkenyl thioether, (C₂₋₁₀)alkynyl thioether, (C₄₋₁₀)carbocyclic thioether;
   (iii) linear or branched, substituted or non-substituted (C₂₋₁₀) NHR₆ or N(R₆)₂, wherein one or both R₆ are independently selected or together form a substituted or non-substituted (C₃₋₁₀)carbocyclic amine;
   (iv) linear or branched, substituted or non-substituted (C₂₋₁₀)alkoxyalkyl, preferably 2-ethoxyethyl, 2-fluorethoxyethyl;
R2 is substituted or non-substituted (3s, 5s, 7s)adamantyl, preferably substituted or non-substituted (3s, 5s, 7s)adamant-1-yl, more preferably 3-substituted (3s, 5s, 7s)adamant-1-yl, wherein the 3-substitutent is preferably selected from the group consisting of hydroxy, amino, -NHR₆, -NH(R₆)₂, wherein each R₆ is selected independently from one another, thio, (C₁₋₁₀)alkyl, (C₂₋₁₀)alkenyl, (C₁₋₁₀)alkinyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, halogen, preferably Cl or F, most preferably R2 is 3-hydroxy- or 3-fluoro(3s, 5s, 7s)adamant-1-yl;
R3 is linear or branched, substituted or non-substituted (C₁₋₁₀)alkyl, (C₂₋₁₀)alkenyl, (C₂₋₁₀)alkynyl, (C₃₋₁₀)carbocycle, preferably (C₃₋₆)cylcoalkyl or a (C₅₋₆)heterocycle having 1 or 2 heteroatoms each independently selected from N, O or S, preferably R3 is (C₁₋₅)alkyl, most preferably methyl, ethyl, propyl;
R4 is H, F, Cl, Br, -CF₃, -CF₂CH₃, cyano, nitro, linear or branched, substituted or non-substituted (C₁₋₁₀)alkyl, (C₂₋₁₀)alkenyl, (C₂₋₁₀)alkynyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, preferably R4 is H;
R5 is H, F, Cl, Br, -CF₃, -CF₂CH₃, cyano, nitro, linear or branched, substituted or non-substituted (C₁₋₁₀)alkyl, (C₂₋₁₀)alkenyl, (C₂₋₁₀)alkynyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, preferably R5 is H;
R₆ is linear or branched, substituted or non-substituted (C₁₋₁₀)alkyl, (C₂₋₁₀)alkenyl, (C₂₋₁₀)alkynyl, (C₃₋₁₀)carbocycle, preferably (C₃₋₆)cylcoalkyl or a (C₅₋₆)heterocycle having 1 or 2 heteroatoms each independently selected from N, O or S;
or R3 and R1 together form a substituted or non-substituted 6-membered heterocyclic ring, wherein X is N or CH, Z is selected from -O-, -NH-, -NR₆-, -S-, substituted or non-substituted-CH₂-, R1 and R3 are independently of one another substituted or non-substituted -CH2-, with the proviso that at least one of R1 or R2 is substituted by
   (i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
   (ii) linear or branched, substituted or non-substituted (C₁₋₁₀)alkyl thioether, (C₂₋₁₀)alkenyl thioether, (C₂₋₁₀)alkynyl thioether, (C₄₋₁₀)carbocyclic thioether;
   (iii) linear or branched, substituted or non-substituted (C₂₋₁₀) NHR₆ or N(R₆)₂, wherein one or both R₆ are independently selected, substituted or non-substituted (C₃₋₁₀)carbocyclic amine;
   (iv) linear or branched, substituted or non-substituted (C₂₋₁₀)alkoxyalkyl, preferably 2-ethoxyethyl, 2-fluorethoxyethyl;
   (v) hydroxy, thiol, amino,
and pharmaceutically acceptable salts or solvates thereof.

In the context of the present invention it is understood that antecedent terms such as linear or branched, substituted or non-substituted indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₂) alkenyl, alkynyl or alkylidene" indicates the group of compounds having 2 to 12 carbons and alkenyl, alkynyl or alkylidene functionality.

The compounds of the present invention are chemically stable and all bind selectively to the CB2 receptor relative to binding to the CB1 receptor. Preferably, the compounds of the invention have an affinity to the CB2 receptor in the nanomolar range, preferably at least 100 nM, more preferably at least 10 nM, most preferably at least less than 5 nM. Preferably, the compounds of the invention have an affinity to the CB2 receptor at least 100, preferably at least 500, more preferably at least 1000, most preferably at least 5000 times higher than their binding affinity for the CB1 receptor. Assays for assessing CB2 and CB1 receptors are common general knowledge in the field of the ECS system and can be found, for example, in Mu et al. (Journal of Neurochemistry, 2013, 126, 616-624) or Pasquini et al. (J. Med. Chem. 2008, 51, 5075-5084) and in Example 2 below.

In formulas I and II A and B are selected independently of each other from -O-, -S- and-NR₆-. In a preferred embodiment the compounds of formula I or II are those, wherein at least one of A and B is -O-. Preferably A and B are both -O-.

In formulas I and II X is -N- or -CH- and Y is selected from -O-, -NH-, -NR₆-, -S-, substituted or unsubstituted -CH₂- or a direct bond. In a preferred embodiment the compounds of formula I or II are those, wherein X is N or Y is -NH-, preferably X is N and Y is -NH- .

In formulas I and II Z is selected from -O-, -NH-, -S-, substituted or unsubstituted -CH₂- or a direct bond. In a preferred embodiment of the compounds of formula I or II Z is -O-. In formulas I and II R4 and R5 are selected independently of each other from H, F, Cl, Br, -CF₃,-CF₂CH₃, cyano, nitro, linear or branched, substituted or non-substituted (C₁₋₁₀)alkyl, (C₂₋₁₀)alkenyl, (C₂₋₁₀)alkynyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S. In a preferred embodiment R4 is H, F, Cl, Br, -CF₃, -CF₂CH₃, cyano, nitro, linear or branched, substituted or non-substituted (C₁₋₄)alkyl, (C₂₋₄)alkenyl, (C₂₋₄)alkynyl, (C₁₋₄)alkoxy, (C₃₋₆)carbocycle, preferably (C₃₋₆)cylcoalkyl or a (C₅₋₆)heterocycle having 1 or 2 heteroatoms each independently selected from N, O or S, most preferably R4 is H. In a more preferred embodiment, at least one of R4 and R5 is H, preferably R4 is H. More preferably both R4 and R5 are H.

In formulas I and II R1 is selected from the group consisting of
(i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
(ii) linear or branched, substituted or non-substituted (C₁₋₁₀)alkyl thioether, (C₂₋₁₀)alkenyl thioether, (C₂₋₁₀)alkynyl thioether, (C₄₋₁₀)carbocyclic thioether;
(iii) linear or branched, substituted or non-substituted (C₂₋₁₀) NHR₆ or N(R₆)₂, wherein one or both R₆ are independently selected or form a substituted or non-substituted (C₃₋₁₀)carbocyclic amine;
(iv) linear or branched, substituted or non-substituted (C₂₋₁₀)alkoxyalkyl, preferably 2-ethoxyethyl, 2-fluorethoxyethyl.

In a preferred embodiment R1 in formulas I and II is selected from the group consisting of
(i) linear or branched, substituted or non-substituted (C₁₋₈)alkyl-, alkenyl-, alkynyl ether, (C₄₋₈)-carbocyclic ether, (C₁₋₄)alkyl-, alkenyl-, alkynyl ether, (C₄₋₅)carbocyclic ether;
(iii) linear or branched, substituted or non-substituted (C₁₋₈) NHR₆ or N(R₆)₂, wherein one or both R₆ are independently selected or form a substituted or non-substituted (C₄₋₈)carbocyclic amine;
(iv) linear or branched, substituted or non-substituted (C₂₋₈)alkoxyalkyl, preferably (C₂₋₆)alkoxyalkyl, more preferably (C₂₋₄)alkoxyalkyl, most preferably 2-ethoxyethyl, 2-fluorethoxyethyl and chloroethoxyethyl.

Without wishing to be bound by theory, it is believed that substituent R1 in the compounds of formulas I and II has a positive impact on EB receptor binding, in particular CB2 receptor selectivity and substantially improves biodistribution of the compounds in the mammalian body and its organs. The improved selectivity together with the improved biodistribution makes the compounds of the present invention excellent candidates for diagnostic and therapeutic applications relating to the ECS in mammals.

In formulas I and II R2 is substituted or unsubstituted (3s, 5s, 7s)adamantyl, preferably substituted or unsubstituted (3s, 5s, 7s)adamant-1-yl, more preferably 3-substituted (3s, 5s, 7s)adamant-1-yl, wherein the 3-substitutent is preferably selected from the group consisting of hydroxy, amino, -NHR₆, -NH(R₆)₂, wherein each R₆ is selected independently from one another, thio, (C₁₋₁₀)alkyl, (C₂₋₁₀)alkenyl, (C₁₋₁₀)alkinyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, halogen, preferably Cl or F, most preferably R2 is 3-hydroxy- or 3-fluoro(3s, 5s, 7s)adamant-1-yl. In a preferred embodiment R2 is 3-substituted (3s, 5s, 7s)adamant-1-yl, wherein the 3-substitutent is preferably selected from the group consisting of hydroxy, amino,-NHR₆, -NH(R₆)₂, wherein each R₆ is selected independently from one another, hydroxythio, (C₁₋₈)-alkyl, alkenyl-, alkinyl-, alkoxy, preferably (C₁₋₄)- alkyl, alkenyl-, alkinyl-, alkoxy, (C₃₋₆)cylcoalkyl, (C₅₋₆)heterocycle having 1 or 2 heteroatoms each independently selected from N, O or S, halogen, preferably Cl or F, most preferably R2 is 3-hydroxy- or 3-fluoro(3s, 5s, 7s)adamant-1-yl.

In formulas I and II R3 is linear or branched, substituted or non-substituted (C₁₋₁₀)alkyl, (C₂₋₁₀)alkenyl, (C₂₋₁₀)alkynyl, (C₃₋₁₀)carbocycle, preferably (C₃₋₆)cylcoalkyl or a (C₅₋₆)heterocycle having 1 or 2 heteroatoms each independently selected from N, O or S, preferably R3 is (C₁₋₅)alkyl, most preferably methyl, ethyl, propyl. In a preferred embodiment R3 is linear or branched, substituted or non-substituted (C₁₋₈)-alkyl, alkenyl, alkynyl, (C₃₋₈)carbocycle, preferably (C₃₋₆)cylcoalkyl or a (C₅₋₆)heterocycle having 1 or 2 heteroatoms each independently selected from N, O or S, more preferably R3 is substituted or unsubstituted (C₁₋₅)alkyl, most preferably methyl, ethyl, propyl.

In formulas I and II R₆ is linear or branched, substituted or non-substituted (C₁₋₁₀)alkyl, (C₂₋₁₀)alkenyl, (C₂₋₁₀)alkynyl, (C₃₋₁₀)carbocycle, preferably (C₃₋₆)cylcoalkyl or a (C₅₋₆)heterocycle having 1 or 2 heteroatoms each independently selected from N, O or S. In a preferred embodiment R₆ is linear or branched, substituted or non-substituted (C₁₋₆)alkyl, alkenyl, alkynyl, preferably (C₁₋₄)alkyl, alkenyl, alkynyl, (C₃₋₆)carbocycle, preferably (C₃₋₆)cylcoalkyl or a (C₅₋₆)heterocycle having 1 or 2 heteroatoms each independently selected from N, O or S.

In formula II R3 and R1 together form a substituted or unsubstituted 6-membered heterocyclic ring, wherein X is N or CH, Z is selected from -O-, -NH-, -NR₆-, -S-, substituted or unsubstituted -CH₂-, R1 and R3 are independently of one another substituted or unsubstituted-CH2-, with the proviso that at least one of R1 or R3 is substituted by
(i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
(ii) linear or branched, substituted or non-substituted (C₁₋₁₀)alkyl thioether, (C₂₋₁₀)alkenyl thioether, (C₂₋₁₀)alkynyl thioether, (C₄₋₁₀)carbocyclic thioether;
(iii) linear or branched, substituted or non-substituted (C₂₋₁₀) NHR₆ or N(R₆)₂, wherein one or both R₆ are independently selected or together form a substituted or non-substituted (C₃₋₁₀)carbocyclic amine;
(iv) linear or branched, substituted or non-substituted (C₂₋₁₀)alkoxyalkyl, preferably 2-ethoxyethyl, 2-fluorethoxyethyl;
(v) hydroxy, thiol, amino.

In a preferred embodiment of formula (II) R3 and R1 together form a substituted or unsubstituted 6-membered heterocyclic ring, wherein X is N or CH, Z is selected from -O-, -NH-,-NR₆-, -S-, substituted or unsubstituted -CH₂-, R1 and R3 are independently of one another substituted or unsubstituted -CH₂-, with the proviso that at least one of R1 or R3 is substituted by
(i) linear or branched, substituted or non-substituted (C₁₋₈)alkyl ether, (C₂₋₈)alkenyl ether, (C₂₋₈)alkynyl ether, (C₄₋₈)carbocyclic ether;
(ii) linear or branched, substituted or non-substituted (C₁₋₈)alkyl thioether, (C₂₋₈)alkenyl thioether, (C₂₋₈)alkynyl thioether, (C₄₋₈)carbocyclic thioether;
(iii) linear or branched, substituted or non-substituted (C₂₋₈) NHR₆ or N(R₆)₂, wherein one or both R₆ are independently selected or together form a substituted or non-substituted (C₃₋₈)carbocyclic amine;
(iv) linear or branched, substituted or non-substituted (C₂₋₈)alkoxyalkyl, preferably (C₂₋₄)alkoxyalkyl, more preferably 2-ethoxyethyl or 2-fluorethoxyethyl;
(v) hydroxy, thiol, amino.

The compounds of the present invention have an improved biodistribution and seem less prone to plasma protein binding, which can be influenced by the lipophilicity of compounds. In a preferred embodiment of the present invention the compounds of formula I and II preferably have a distribution coefficient (logD) in 1-octanol, phosphate buffer at pH 7.4 of at most 3.5, preferably at most 3.0, more preferably at most 2.8, most preferably at most 2.

In a more preferred embodiment of the present invention the compounds of formula I and II are those, wherein A and B are both -O-, X is N or -CH-, Y is -O- or -NH-, Z is -O-, R4 and R5 are both H,
R1 is -(C(R7)₂)ₙ-M-C(R8)₂)ₘ-C(R9)₃, wherein n is 1 to 4 and m is 0 to 4, M is -O-, NH, NR₆, or -S-, and each of R7, R8 and R9 is independently selected from H and halogen, preferably H, Cl and F;
R2 is 3-substituted (3s, 5s, 7s)adamant-1-yl, wherein the 3-substitutent is preferably selected from the group consisting of hydroxy, amino, -NHR₆, -NH(R₆)₂, wherein each R₆ is selected independently from one another, thio, (C₁₋₄)-alkyl, halogen, preferably Cl or F;
R3 is linear or branched, substituted or non-substituted (C₁₋₅)alkyl, (C₂₋₅)alkenyl, (C₂₋₅)alkynyl, (C₃₋₆)carbocycle, preferably (C₃₋₆)cylcoalkyl or a (C₅₋₆)heterocycle having 1 or 2 heteroatoms each independently selected from N, O or S, preferably R3 is (C₁₋₅)alkyl, most preferably methyl, ethyl, propyl.

In a most preferred embodiment the compounds of the present invention are selected from the group consisting of
(i) *N*-(1-adamantyl)-1-butyl-8-methoxy-4-oxo-1,4-dihydroquinoline-3-carboxamide
(ii) *N*-(*tert*-butyl)-1-butyl-8-methoxy-4-oxo-1,4-dihydroquinoline-3-carboxamide
(iii) 1-butyl-*N*-cyclopentyl-8-methoxy-4-oxo-1,4-dihydroquinoline-3-carboxamide
(iv) 1-butyl-*N*-(cyclopropylmethyl)-8-methoxy-4-oxo-1,4-dihydroquinoline-3-carboxamide
(v) *N*-(*tert*-butyl)-1-(3-fluoropropyl)-8-methoxy-4-oxo-1,4-dihydroquinoline-3-carboxamide
(vi) *N*-(1-adamantyl)-1-(2-ethoxyethyl)-8-methoxy-4-oxo-1,4-dihydroquinoline-3-carboxamide
(vii) 1-(2-ethoxyethyl)-8-methoxy-4-oxo-*N*-phenethyl-1,4-dihydroquinoline-3-carboxamide
(viii) 1-(2-ethoxyethyl)-*N*-(3-hydroxyadamantan-1-yl)-8-methoxy-4-oxo-1,4-dihydroquinoline-3-carboxamide
(ix) 1-(2-ethoxyethyl)-*N*-(3-fluoroadamantyl)-8-methoxy-4-oxo-1,4-dihydroquinoline-3-carboxamide
(x) *N*-(1-adamantanyl)-1-(2-ethoxyethyl)-8-(2-fluoroethoxy)-4-oxo-1,4-dihydroquinoline-3-carboxamide
(xi) *N*-(1-adamantyl)-1-(2-(2-fluoroethoxy)ethyl)-8-methoxy-4-oxo-1,4-dihydroquinoline-3-carboxamide.

### DEFINITIONS

In all compounds disclosed herein, in the event that the nomenclature conflicts with the structure, it shall be understood that the compound is defined by the structure.

The invention includes all compounds described herein containing one or more asymmetric carbon atoms that may occur as racemates and racemic mixtures, single enantiomers, diastereoisomeric mixtures and individual diastereoisomers. All such isomeric forms of these compounds are expressly included in the present invention. Each stereogenic carbon may be in the R or *S* configuration or a combination of configurations. Some of the compounds of the general formulas (I) and (II) disclosed herein can exist in more than one tautomeric form. The present invention includes all such tautomers.

All terms as used herein shall be understood by their ordinary meaning as known in the art.

The term "heteroatom" as used herein shall be understood to mean atoms other than carbon and hydrogen such as and preferably O, N, S and P.

The terms alkyl, alkenyl, alkynyl, alkylidene, etc. shall be understood as encompassing linear as well as branched forms of carbon-containing chains where structurally possible. In these carbon chains one or more carbon atoms can be optionally replaced by heteroatoms, preferably by O, S or N. If N is not substituted it is NH. The heteroatoms may replace either terminal or internal carbon atoms within a linear or branched carbon chain. Such groups can be substituted as herein described by groups such as oxo to result in definitions such as but not limited to alkoxycarbonyl, acryl, amido and thioxo.

The term "carbocycle" shall be understood to mean an aliphatic hydrocarbon radical containing from 3 to 20, preferably from 3 to 12 carbon atoms, more preferably 5 or 6 carbon atoms. Carbocylces include hydrocarbon rings containing from 3 to 10 carbon atoms. These carbocycles may be either aromatic or non-aromatic systems. The non-aromatic ring systems may be mono or polyunsaturated. Preferred carbocycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, and benzocycloheptenyl. Certain terms for cycloalkyl such as cyclobutanyl and cyclobutyl shall be used interchangeably.

The term "cycloalkyl" shall be understood to mean aliphatic hydrocarbon-containing rings having from 3 to 12 carbon atoms. These non-aromatic ring systems may be mono- or polyunsaturated, i.e. the term encompasses cycloalkenyl and cycloalkynyl. The cycloalkyl may comprise heteroatoms, preferably O, S or N, and be substituted or non-substituted. Preferred and non-limiting cycloalkyls include cyclopropyl, cyclobutyl, cyclopentyl, cyclopentenyl, cyclohexyl, cyclohexenyl, cycloheptanyl, cycloheptenyl, benzocyclobutanyl, benzocycloheptanyl und benzocycloheptenyl.

The term "heterocyclic" refers to a stable non-aromatic, preferably 3 to 20 membered, more preferably 3 - 12 membered, most preferably 5 or 6 membered, monocyclic or multicyclic, preferably 8 - 12 membered bicyclic, heteroatom-containing cyclic radical, that may be either saturated or unsaturated. Each heterocycle consists of carbon atoms and one or more, preferably 1 to 4 heteroatoms chosen from nitrogen, oxygen and sulphur. The heterocyclic residue may be bound to the remaining structure of the complete molecule by any atom of the cycle, which results in a stable structure. Exemplary heterocycles include but are not limited to pyrrolidinyl, pyrrolinyl, morpholinyl, thiomorpholinyl, thiomorpholinyl sulfoxide, thiomorpholinyl sulfone, dioxalanyl, piperidinyl, piperazinyl, tetrahydrofuranyl, 1-oxo-λ4-thiomorpholinyl, 13-oxa-11-aza-tricyclo[7.3.1.0-2,7]tridecy-2,4,6-triene, tetrahydropyranyl, 2-oxo-2H-pyranyl, tetrahydrofuranyl, 1,3-dioxolanone, 1,3-dioxanone, 1,4-dioxanyl, 8-oxa-3-aza-bicyclo[3.2.1]-octanyl, 2-oxa-5-aza-bicyclo[2.2.1]heptanyl, 2-thia-5-aza-bicyclo[2.2.1]heptanyl, piperidinonyl, tetrahydro-pyrimidonyl, pentamethylene sulphide, pentamethylene sulfoxide, pentamethylene sulfone, tetramethylene sulphide, tetramethylene sulfoxide and tetramethylene sulfone.

The term "aryl" as used herein shall be understood to mean an aromatic carbocycle or heteroaryl as defined herein. Each aryl or heteroaryl unless otherwise specified includes its partially or fully hydrogenated derivative. For example, quinolinyl may include decahydroquinolinyl and tetrahydroquinolinyl; naphthyl may include its hydrogenated derivatives such as tetrahydronaphthyl. Other partially or fully hydrogenated derivatives of the aryl and heteroaryl compounds described herein will be apparent to one of ordinary skill in the art. Naturally, the term encompasses aralkyl and alkylaryl, both of which are preferred embodiments for practicing the compounds of the present invention. For example, the term aryl encompasses phenyl, indanyl, indenyl, dihydronaphthyl, tetrahydronaphthyl, naphthyl and decahydronaphthyl.

The term "heteroaryl" shall be understood to mean an aromatic C₃-C₂₀, preferably 5 - 8 membered monoxyclic or preferably 8 - 12 membered bicyclic ring containing 1 - 4 heteroatoms such as N, O and S. Exemplary heteroaryls comprise aziridinyl, thienyl, furanyl, isoxazolyl, oxazolyl, thiazolyl, thiadiazolyl, tetrazolyl, pyrazolyl, pyrrolyl, imidazolyl, pyridinyl, pyrimidinyl, pyrazinyl, pyridazinyl, pyranyl, quinoxalinyl, indolyl, benzimidazolyl, benzoxazolyl, benzothiazolyl, benzothienyl, quinolinyl, quinazolinyl, naphthyridinyl, indazolyl, triazolyl, pyrazolo[3,4-*b*]pyrimidinyl, purinyl, pyrrolo[2,3-*b*]pyridinyl, pyrazole[3,4-*b*]pyridinyl, tubercidinyl, oxazo[4,5-*b*]pyridinyl and imidazo[4,5-*b*]pyridinyl.

Terms which are analogues of the above cyclic moieties such as aryloxy or heteroaryl amine shall be understood to mean an aryl, heteroaryl, heterocycle as defined above attached to its respective group.

As used herein, the terms "nitrogen" and "sulphur" include any oxidized form of nitrogen and sulphur and the quaternized form of any basic nitrogen as long as the resulting compound is chemically stable. For example, for an -S-C₁₋₆ alkyl radical shall be understood to include -S(O)-C₁₋₆alkyl and -S(O)₂-C₁₋₆alkyl.

The compounds of the invention are only those which are contemplated to be 'chemically stable' as will be appreciated by those skilled in the art. For example, compounds having a 'dangling valency' or a 'carbanion' are not compounds contemplated by the inventive disclosed herein.

Compound of the present invention have utility in the diagnosis of ECS-, in particular CB2 receptor-related diseases, where quantitative and biodistribution data on the CB2 receptor can be interpreted to differentiate diseases from the healthy state in a mammal. For diagnostic purposes the compound is preferably marked for easy identification and quantification, for example, radiolabeled. The compounds of the present invention are preferably radiolabelled by an isotope selected from the group consisting of non-metallic position emitting isotopes and ¹¹C, ¹⁸F. In a preferred embodiment the compounds of formulas I and II are radiolabeled in any of R₁, R₂ or R₃, preferably in R₃ or R₂, most preferably in R₃.

In a further aspect, the present invention is directed to the use of a compound of formulas I or II for determining cannabinoid receptor 2 (CB2)-selective receptor localization and/or density, preferably in the central nervous system (CNS), the peripheral nervous system (PNS), heart, liver, gastrointestinal tract, spleen, pancreas, kidney, testis, ovary and/or the prostate.

In a preferred embodiment, the present invention refers to the use of a compound of formulas I and II for determining cannabinoid receptor 2 (CB2)-upregulation in microglia.

In a very preferred embodiment, the present invention refers to a compound according to formulas I and II for use in the diagnosis, prophylaxis and/or therapy of CB2 receptor-related diseases, preferably selected from the group of CB2 receptor-related diseases consisting of cardiovascular disease, myocardial infarction, ischemia reperfusion injury, heart failure, cardiomyopathies, atherosclerosis, restenosis, stroke, spinal cord injury, cirrhotic cardiomyopathy, septic shock by live bacteria, hepatic ischaemia reperfusion injury, obesity, non-alcoholic fatty liver disease, diabetes, diabetic complications, obesity/metabolic syndrome; liver, gastrointestinal and skin diseases; liver fibrosis, cirrhosis, alcohol-induced liver injury, pancreatitis, inflammatory bowel disease, colitis, diverticulitis, nephropathy, neurodegenerative/neuroinflammatory disorders, in particular multiple sclerosis (MS), Alzheimer's disease (AD), Parkinson's disease (PD) and Huntington's disease (HD); spinal cord injury, pain; psychiatric disorders, in particular anxiety and depression schizophrenia; rheumatoid arthritis, cachexia, cancer, chemotherapy-induced nausea and vomiting.

In a most preferred aspect the present invention is directed to compounds of formula I and II for use in the diagnosis, prophylaxis and/or therapy of neuroinflammatory and neurodegenerative diseases, preferably diseases selected from the group consisting of multiple sclerosis (MS), amyotrophic lateral sclerosis (ALS), Alzheimer's disease (AD) and Parkinson's disease (PD).

In a most preferred aspect the present invention is directed to compounds of formula I and II for the prophylaxis and/or therapy of pain, preferably hyperalgesia.

In a preferred embodiment one or more compounds of the present invention are used for preparing a medicament or diagnostic composition for the diagnosis, treatment and/or prevention of an ECS-, preferably a CB2-related disease, more preferably a disease or condition mentioned above.

A further aspect of the present invention concerns pharmaceutical or diagnostic compositions, comprising as active or diagnostic substance one or more compounds of the present invention or pharmaceuticclly acceptable derivatives or prodrugs thereof, optionally combined with conventional excipients and/or carriers.

### DIAGNOSTIC AND MEDICAL USE, DIAGNOSTIC AND PHARMACEUTICAL COMPOSITIONS

The invention includes pharmaceutically acceptable derivatives of the compounds of formulae (I) and (II). A "pharmaceutically acceptable derivative" refers to any pharmaceutically acceptable salt or ester or any other compound which, upon administration to a patient, is capable of providing (directly or indirectly) a compound of the invention, or a pharmacologically active metabolite or pharmacologically active residue thereof. A pharmacologically active metabolite shall be understood to mean any compound of the invention capable of being metabolized enzymatically or chemically. This includes, for example, hydroxylated or oxidized derivative compounds of the formula (I) and (II). Preferred embodiments relate to pharmaceutically acceptable derivatives of compounds of formulas (I) and (II) that are hydrates.

Pharmaceutically acceptable salts include those derived from pharmaceutically acceptable inorganic and organic acids and bases. Examples of suitable acids include hydrochloric, hydrobromic, sulphuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal (e.g. magnesium), ammonium and N-(C₁-C₄ alkyl)₄⁺ salts.

In addition, the scope of the invention also encompasses prodrugs of compounds of the formulas (I) and (II). Prodrugs include those compounds that, upon simple chemical transformation, are modified to produce compounds of the invention. Simple chemical transformations include hydrolysis, oxidation and reduction. Specifically, when a prodrug is administered to a patient, the prodrug may be transformed into a compound disclosed hereinabove, thereby imparting the desired pharmacological effect.

The compounds of the invention have demonstrated a selective and high affinity binding to the EC2 receptor and do not affect the CB2 receptor. And they demonstrate an effective biodistribution in mammals.

Hence, in a further aspect the present invention is directed to the use of one or more compounds according to the invention for preparing a medicament or diagniostoic means. Preferably, the compounds of the invention are used for preparing a medicament or diagnostic composition for the diagnosis,treatment and/or prevention of ECS-related diseases, preferably neurodegenerative diseases, preferably selected from MS, ALS, AD and PD.

In the above respect the present invention also relates to a pharmaceutical or diasgnostic composition, comprising as active substance one or more compounds according to the invention or pharmaceutically acceptable derivatives or prodrugs thereof, optionally combined with conventional excipients and/or carriers.

### METHODS OF USE

For diagnostic and therapeutic use the compounds of the invention may be administered in any conventional dosage form in any conventional manner. Routes of administration include, oral, intravenous, intramuscular and subcutaneous injections. The preferred modes of administration are oral and intravenous.

The compounds may be administered alone or in combination with adjuvants that enhance stability of the compounds, facilitate administration of pharmaceutical compositions containing them in certain embodiments, provide increased dissolution or dispersion, increase activity, provide adjunct therapy, and the like, including other active ingredients. Advantageously such combination therapies utilize lower dosages of the conventional therapeutics, thus avoiding possible toxicity and adverse side effects incurred when those agents are used as monotherapies. The above described compounds may be physically combined with conventional therapeutics or other adjuvants into a single pharmaceutical composition. Reference in this regard may be made to Cappola et al.: U.S. patent application no. 09/902,822, PCT/US 01/21860 und US provisional application no. 60/313,527, each incorporated by reference herein in their entirety. Advantageously, the compounds may then be administered together in a single dosage form. In some embodiments, the pharmaceutical compositions comprising such combinations of compounds contain at least about 5 %, but more preferably at least about 20 %, of a compound of formula (I) or (II) (w/w) or a combination thereof. The optimum percentage (w/w) of a compound of the invention may vary and is within the purview of those skilled in the art. Alternatively, the compounds may be administered separately (either serially or in parallel). Separate dosing allows for greater flexibility in the dosing regime.

As mentioned above, dosage forms of the compounds described herein include pharmaceutically acceptable carriers and adjuvants known to those of ordinary skill in the art. Methods for preparing such dosage forms are known (see, for example, H. C. Ansel and N. G. Popovish, Pharmaceutical Dosage Forms and Drug DeliverySystems, 5th ed., Lea and Febiger (1990)). Dosage levels and requirements are well-recognized in the art and may be selected by those of ordinary skill in the art from available methods and techniques suitable for a particular patient. In some embodiments, dosage levels range from about 1-100 mg/dose for a 70 kg patient. Although one dose per day may be sufficient, up to 5 doses per day may be given. For oral doses, up to 2000 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician.

In the following, the invention will be illustrated by way of specific examples, none of which are to be interpreted as limiting the scope of the claims as appended.

### Figures

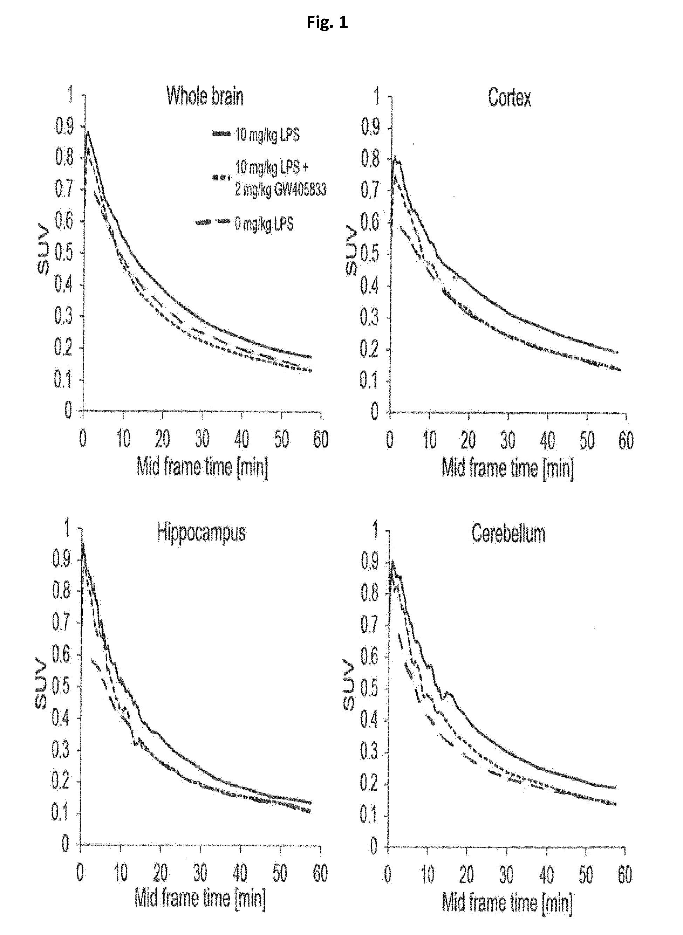

**Fig. 1** shows time-activity curves of [¹¹C]RS-016 (*N*-(1-adamantyl)-1-(2-ethoxyethyl)-8-methoxy-4-oxo-1,4-dihydroquinoline-3-carboxamide) in whole brain, cortex, hippocampus, and cerebellum 5 days after application of 0 or 10 mg/kg LPS (solid lines) and under blocking conditions with 2 mg/kg GW405833 (1-(2,3-Dichlorobenzoyl)-5-methoxy-2-methyl-(3-(morpholin-4-yl)ethyl)-1H-indole, Sigma-Aldrich, Switzerland).
30 min prior injection of [¹¹C]RS-016 (dashed line). For each condition n = 3.

### Examples

### Example 1-Synthesis of compounds

The synthesis of the compounds with the general structure of formula I can, for example, be accomplished according to the following schemes.

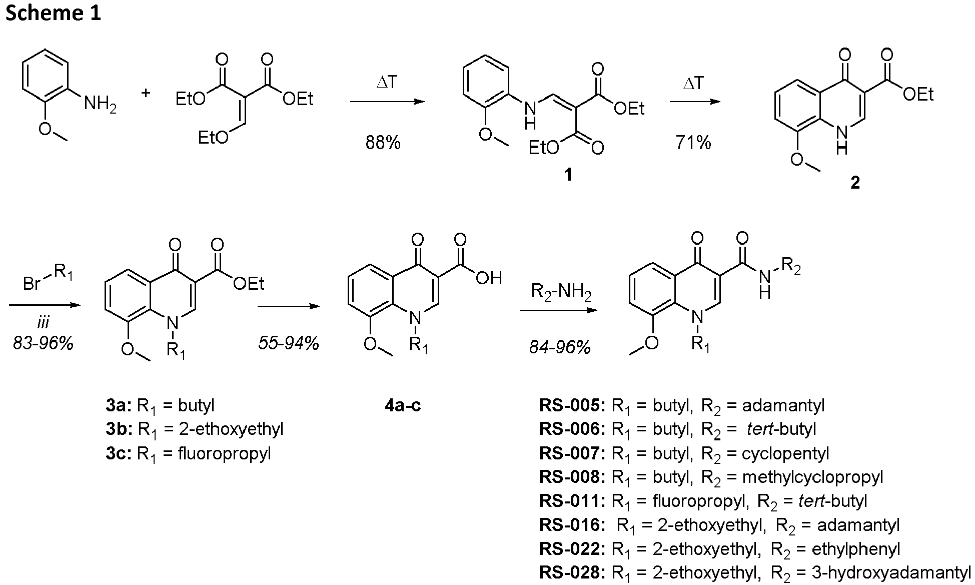

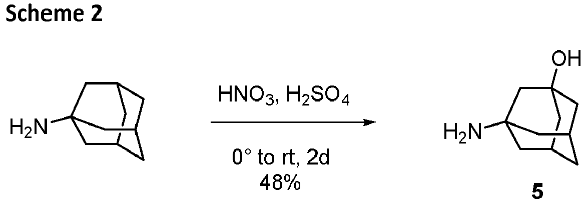

**Synthesis of diethyl 2-(((2-methoxyphenyl)amino)methylene)malonate (1).** To 2-anisidine (6.36 g, 51.6 mmol) was added diethyl 2-(ethoxymethylene)malonate (11.17 g, 51.6 mmol). The mixture was stirred and heated in an oil bath to 110 °C and stirred 1h. After cooling to rt, the crude mixture was recrystallized from hexane (50 mL) to give 1 in a yield of 88%. HRMS calcd for C₁₅H₁₉NNaO₅ 316.1155, found 316.1164.

**Synthesis of ethyl 8-methoxy-4-oxo-1,4-dihydroquinoline-3-carboxylate (2).** To 1 (13.00 g, 44.3 mmol) was added diphenylether (70 mL). The reaction mixture was heated to 250 °C for 1h. After cooling to rt, the mixture was filtrated and the precipitates washed with diphenylether (10 mL) and hexane (3 x 10 mL). After recrystallization from ethanol (150 mL), the precipitates were collected by filtration and dried under reduced pressure to give 2 in a yield of 70%. HRMS calcd for C₁₃H₁₃NNaO₄ 270.0737, found 270.0743.

**Representative procedure for amine alkylation. Synthesis of ethyl 1-butyl-8-methoxy-4-oxo-1,4-dihydroquinoline-3-carboxylate (3a).** To a solution of **2** (0.28 g, 1.132 mmol) in DMF (5 mL) was added potassium carbonate (0.438 g, 3.17 mmol) and 1-bromobutane (0.340 ml, 3.17 mmol). The mixture was heated to 90 °C for 4h, then cooled to rt and aq. HCl (0.2M, 50 mL) was added. The mixture was extracted with DCM (3 x 5 mL) and the combined organic layers dried over MgSO₄. Solvents were removed under reduced pressure and the residue was purified over silica gel using hexane:EtOAc (1:1) to give 3a (0.33 g, 96 %).

Ethyl 1-(2-ethoxyethyl)-8-methoxy-4-oxo-1,4-dihydroquinoline-3-carboxylate **(3b)** and Ethyl 1-(3-fluoropropyl)-8-methoxy-4-oxo-1,4-dihydroquinoline-3-carboxylate **(3c)** were similarly prepared.

**Representative procedure for hydrolysis. Synthesis of 1-butyl-8-methoxy-4-oxo-1,4-dihydroquinoline-3-carboxylic acid (4a).** Aq. NaOH (10%, 30 mL) was added to **3a** (0.33 g, 1.088 mmol). The mixture was heated to reflux for 2h. After cooling to rt, pH was adjusted to ∼2 using conc. HCl and the precipitatets were collected and washed with water and diethyleter. Recristallization from ∼50 ml EtOH gave **4a** (0.278 g, 1.010 mmol, 93 % yield). 1-(2-ethoxyethyl)-8-methoxy-4-oxo-1,4-dihydroquinoline-3-carboxylic acid **(4b)** and 1-(3-fluoropropyl)-8-methoxy-4-oxo-1,4-dihydroquinoline-3-carboxylic acid **(4c)** were in analogy to compound **4a.**

**Synthesis of 3-hydroxy-1-aminoadamantane (5).** To an ice-cooled mixture of sulfuric acid 96% (12.33 mL, 231 mmol) and nitric acid 65% (1.2 mL, 26.5 mmol) was added 1-aminoadamantan HCl (1g, 6.61 mmol) portionwise. The mixture was stirred at rt for 2 days. Ice-water (6 mL) was added to the reaction and solution was placed in an ice-water bath and allowed to stir for 30 minutes. KOH (35 g, 0.62 mol) was added in small portions over 1 h. During this addition, the reaction was never allowed to exceed 80 °C. The resulting white paste was mixed with DCM (300 mL) and vigorously stirred for 1 h. After filtration the organic layer is separated and solvents were removed under reduced pressure to provide **5** (527 mg, 3.15 mmol) in 48% yield as a white solid. HRMS calcd for C₁₀H₁₈NO 168.1383, found 168.1380.

**Representative procedure for amide coupling. Synthesis of *N*-(1-adamantyl)-1-butyl-8-methoxy-4-oxo-1,4-dihydroquinoline-3-carboxamide (RS-005).** To a dispersion of **3a** (206 mg, 0.748 mmol) in DMF (10 mL) was added DIPEA (0.392 ml, 2.245 mmol) and the solution was stirred at rt for 30 min. HBTU (568 mg, 1.497 mmol) was added portion wise, followed by the addition of 1-aminoadamantane (0.136 g, 0.898 mmol). The mixture was stirred for 4h at RT. The reaction was diluted with EtOAc (60 mL) and washed with water (3 x 10 mL), once with diluted HCl (0.5M, 10 mL) and again with water (10 mL) and brine (15 mL). Solvents were removed under reduced pressure and the residue was purified with flash chromatography using hexane:EtOAc (10:1 to 2:1) to give **RS-005** (263 mg, 0.644 mmol, 86 % yield). HRMS calcd for C₂₅H₃₃N₂O₃ 409.2486, found 409.2492.

***N*-(*tert*-butyl)-1-butyl-8-methoxy-4-oxo-1,4-dihydroquinoline-3-carboxamide (RS-006).** HRMS calcd for C₁₉H₂₇N₂O₃ 331.2016, found 331.2018.

**1-butyl-*N*-cyclopentyl-8-methoxy-4-oxo-1,4-dihydroquinoline-3-carboxamide (RS-007).** HRMS calcd for C₂₀H₂₇N₂O₃ 343.2016, found 343.2016.

**1-butyl-*N*-(cyclopropylmethyl)-8-methoxy-4-oxo-1,4-dihydroquinoline-3-carboxamide (RS-008).** HRMS calcd for C₁₉H₂₅N₂O₃ 329.1860, found 329.1859.

***N*-(tert-butyl)-1-(3-fluoropropyl)-8-methoxy-4-oxo-1,4-dihydroquinoline-3-carboxamide (RS-011).** HRMS calcd for C₁₈H₂₄FN₂O₃ 335.1765, found 335.1763.

***N*-(1-adamantyl)-1-(2-ethoxyethyl)-8-methoxy-4-oxo-1,4-dihydroquinoline-3-**carboxamide (RS-016). HRMS calcd for C₂₅H₃₄N₂O₄ 425.2436, found 425.2436.

**1-(2-ethoxyethyl)-8-methoxy-4-oxo-*N*-phenethyl-1,4-dihydroquinoline-3-carboxamide (RS-022).** HRMS calcd for C₂₃H₂₇N₂O₄ 395.1965, found 395.1964.

**1-(2-ethoxyethyl)-*N*-(3-hydroxyadamantan-1-yl)-8-methoxy-4-oxo-1,4-dihydroquinoline-3-carboxamide (RS-028).** HRMS calcd for C₂₅H₃₃N₂O₅ 441.2384, found 441.2382.

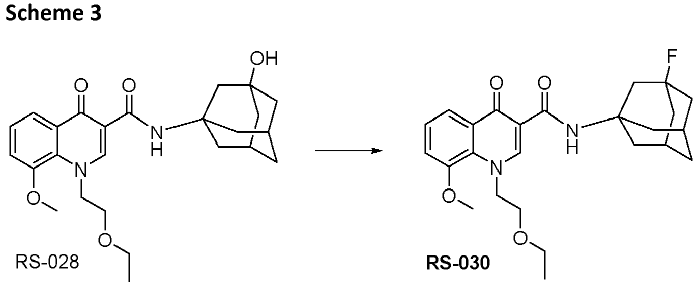

**Synthesis of 1-(2-ethoxyethyl)-*N*-(3-fluoroadamantyl)-8-methoxy-4-oxo-1,4-dihydroquinoline-3-carboxamide (RS-030).** To a -78 °C cold solution of 1-(2-ethoxyethyl)-N-((1*r*,3*s*,5*R*,7*S*)-3-hydroxyadamantan-1-yl)-8-methoxy-4-oxo-1,4-dihydroquinoline-3-carboxamide **(RS-028)** (10 mg, 0.023 mmol) in DCM (0.25 mL) was added DAST (6 µl, 0.045 mmol). The mixture was allowed to warm to RT and stirred for 1 hour. Ice water (5 mL) was added and the reaction was extracted with DCM (3 x 2mL), washed with brine, dried over MgSO4 and solvents were removed under reduced pressure. Crude was purified over silica gel using DCM:MeOH (50:1) to give **RS-030.** HRMS calcd for C₂₅H₃₂FN₂O₄ 443.2341, found 443.2340.

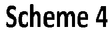

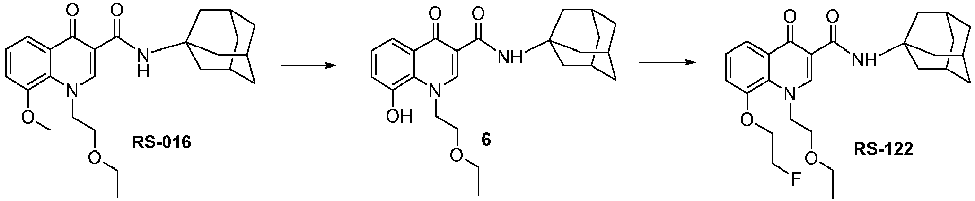

**Synthesis of *N*-(1-adamantyl)-1-(2-ethoxyethyl)-8-hydroxy-4-oxo-1,4-dihydroquinoline-3-carboxamide (6).** To a solution of **RS-016** (202mg, 0.476 mmol) in DMF (5 mL) was added lithium chloride (303 mg, 7.14 mmol). The mixture was heated to reflux overnight. After cooling to rt, EtOAc (60 mL) was added and the mixture was washed with 0.2M HCl (3 x 10 mL) and brine (15 mL). The combined organic layers were dried over MgSO₄ and solvents were removed under reduced pressure. HPLC purification over a C18 column using 0.1% TFA in water and acetonitrile (CH₃CN) 30:70 gave the desired product 6 (37 mg, 0.090 mmol, 20% yield). HRMS calcd for C₂₄H₃₁N₂O₄ 411.2278, found 411.2281.

**Synthesis of *N*-(1-adamantanyl)-1-(2-ethoxyethyl)-8-(2-fluoroethoxy)-4-oxo-1,4-dihydro-quinoline-3-carboxamide (RS-122).** To a solution of **6** (20 mg, 0.05 mmol) and cesium carbonate (24 mg, 0.075 mmol) in DMF (1 mL) was added 2-fluoroethyl 4-methylbenzenesulfonate (13 µL, 0.075 mmol). The mixture was stirred at RT for 24h. The mixture was diluted with aq. HCl (0.2 M, 30 mL) and extracted with DCM (3 x 5 mL). The combined organic layers were washed with brine (20 mL) and dried over MgSO₄. Solvents were removed under reduced pressure and the residue purified over silica gel using DCM:MeOH (100:1) to give **RS-122** (17.5mg, 0.038 mmol, 79 % yield). HRMS calcd for C₂₆H₃₄FN₂O₄ 457.2497, found 457.2495.

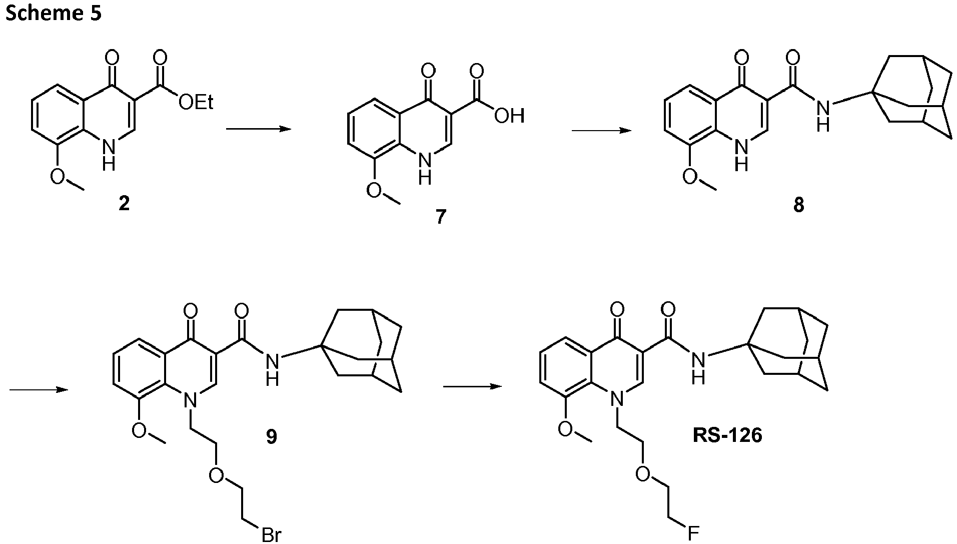

**Synthesis of 8-methoxy-4-oxo-1,4-dihydroquinoline-3-carboxylic acid (7).** To **2** (2.00 g, 8.09 mmol) was added NaOH 10% (140 mL). The mixture was heated to reflux for 3h. The pH was adjusted to ∼2 using conc. HCl and the precipitates were collected and washed with water (15 mL) and petrolether 60/90 (20 mL). The residue was taken up in EtOH (150 mL) and heated to reflux for 30 min. After slow cooling down, the mixture was filtrated and the residue dried in vacuo to give **7** as a slight grey powder in a yield of 94 %.

**Synthesis of *N*-(adamantan-1-yl)-8-methoxy-4-oxo-1,4-dihydroquinoline-3-carboxamide (8)** To RS-009 (219 mg, 1 mmol) in DMF (Volume: 8 ml) was added DIPEA (0.524 ml, 3.00 mmol) and the mixture is stirred at RT for 30 min. HBTU (758 mg, 2.000 mmol) was added portionwise and finally, 1-aminoadamantane (181 mg, 1.200 mmol) was added. The mixture is stirred for 3h at RT. The mixture was diluted with EtOAc (50 mL) and washed with water (3 x 15 mL), once with diluted HCl (15 ml 0.5M) and again with water (15 mL) and then brine (20 mL). EtOAc was evaporated under reduced pressure and the residue was purified with flash chromatography using hexane/EtOAc to give pure RS-015 (330 mg, 0.936 mmol, 94 % yield).

**Synthesis of *N*-(adamantan-1-yl)-1-(2-(2-bromoethoxy)ethyl)-8-methoxy-4-oxo-1,4-dihydroquinoline-3-carboxamide (9).** To a solution of RS-015 (100mg, 0.284 mmol) in DMF (Volume: 2 ml) were added cesium carbonate (137 mg, 0.422 mmol), and 1-bromo-2-(2-bromoethoxy)ethane (53 µL, 0.422 mmol). The mixture was heated under nitrogen to 90 °C for 3h. After cooling to RT, the mixture was poured into ice-water (50 mL) and extracted with DCM (3 x 10 mL), washed with brine (15 mL) and dried over MgSO₄. Solvents were removed under reduced pressure and the residue was purified over silica gel using hexane:EtOAc (1:1) to give pure RS-125 (65mg, 0.129 mmol, 45.9 % yield). HRMS calcd for C₂₅H₃₂BrN₂O₄ 503.1540, found 503.1539.

**Synthesis of *N*-(1-adamantyl)-1-(2-(2-fluoroethoxy)ethyl)-8-methoxy-4-oxo-1,4-dihydro-quinoline-3-carboxamide (RS-126).** To potassium fluoride (9.23 mg, 0.159 mmol) and Kryptofix (59.8 mg, 0.159 mmol) was added ACN (1 mL) and the mixture is heated to 90 °C. 9 (20 mg, 0.040 mmol) was added and the reaction was stirred and heated for 3h. After cooling to RT, water (25 mL) was added and the mixture was extracted with EtOAc (3 x 5 mL). The combined organic layers were dried over MgSO4 and solvents were removed under reduced pressure. The residue was purifed over silica gel using Hex:EtOAc (3:2 to 1:2) to give RS-126 (4mg, 9.04 µmol, 23 % yield). HRMS calcd for C25H32FN2O4 (M+H) 443.2341, found 443.2340.

### Example 2 -In vitro binding assay

The following test was carried out in order to determine the binding affinity of the compounds of formula I towards CB2 and CB1:

Competitive binding reactions were initiated by the addition of a membrane preparation obtained from CHO-K1 cells stably transfected with human CB1 and CB2, respectively, from PerkinElmer (0.5 µg/tube for hCB₁ and hCB₂) into incubation tubes. As displacer, 1.4 nM [³H]CP-55,940 (PerkinElmer) was used and 6 to 10 concentrations (ranging from 1 pM to 10 µM) of displacing ligand (compound to test) in assay buffer (50 mM TRIS, 1 mM EDTA, 3 mM MgCl₂ and 0.05% bovine serum albumin, pH adjusted to 7.4) were added. Nonspecific binding was defined by the presence of 5 µM WIN-55212-2. After incubation at 30 °C for 90 min reactions were terminated by the addition of 3 mL ice cold assay buffer followed by rapid vacuum filtration through a Whatman GF/C filter (pre-soaked for 2 h in 0.05% polyethylenimine in water) and washed twice with 3 mL ice cold assay buffer. The bound activity was counted in a Beckman LS 6500 Liquid Scintillation Counter after adding 3 mL scintillation cocktail (Ultima Gold, Perkin Elmer) and thorough shaking. For each mean value three experiments, each in triplicates, were performed. *K*ᵢ values were determined with the equation from Cheng-Prusoff. For calculations, K_{D} values of 0.14 and 0.11 nM from PerkinElmer were used for [3H]CP-55,940 binding to hCB1 and hCB2 receptors, respectively.
Results obtained for representative compounds of the invention are given in the following table:

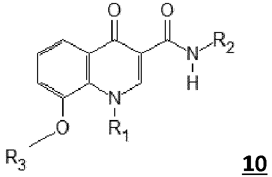

| Example | R₁ | R₂ | R₃ | clogP | K*ᵢ* CB₂ [nM] | K*ᵢ* CB₁ [nM] |
|---|---|---|---|---|---|---|
| KD2* | 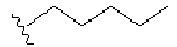 | 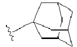 | 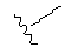 | 4.82 | 1.7 ± 2.0 | >10'000 |
| RS-005 | 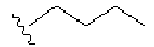 | 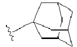 | 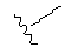 | 4.29 | 3.3 ± 0.2 | >10'000 |
| RS-006 | 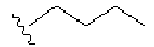 | 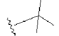 | 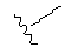 | 2.87 | 8.2 ± 5.5 | >10'000 |
| RS-007 | 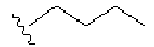 | 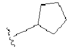 | 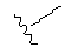 | 3.10 | 19.7 ± 8.8 | >10'000 |
| RS-008 | 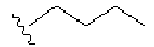 | 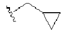 | 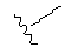 | 2.60 | 78.8 ± 33.1 | >10'000 |
| RS-011 | 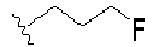 | 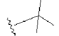 | 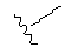 | 1.76 | 750 ± 780 | >10'000 |
| RS-016 | 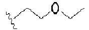 | 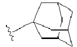 | 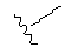 | 3.0 | 0.7 ± 0.6 | >10'000 |
| RS-022 | 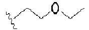 | 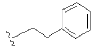 | 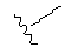 | 2.46 | 360 ± 240 | >10'000 |
| RS-028 | 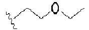 | 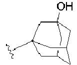 | 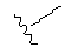 | 1.62 | 0.8 ± 0.8 | >10'000 |
| RS-030 | 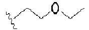 | 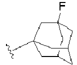 | 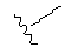 | 2.56 | 2.1 ± 1.5 | >10'000 |
| RS-122 | 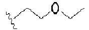 | 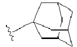 | 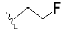 | 3.27 | 72 ± 102 | >10'000 |
| RS-126 | 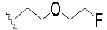 | 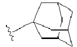 | 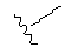 | 2.74 | 1.2 ± 0.8 | >10'000 |

| | | | | | | |
|---|---|---|---|---|---|---|
| * reference compound disclosed by Pasquini et al. (Journal of Medicinal Chemistry, 2011, 54: 5444-5453) | | | | | | |

### Example 3 - Radiolabelling of compounds for PET imaging

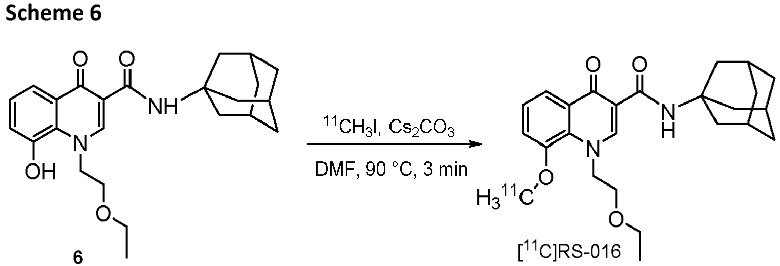

[¹¹C]CO₂ was produced via the 14N(p, *α*)¹¹C nuclear reaction by bombardment of nitrogen gas fortified with 0.5% oxygen using a Cyclone 18/9 cyclotron (18-MeV; IBA, Belgium). After reduction over a supported nickel catalyst to [¹¹C]CH₄ and subsequent gas phase iodination, [¹¹C]CH₃I was bubbled through a mixture of precursor 6 (1 mg) and cesium carbonate (5 mg) in DMF (0.6 mL). The mixture was heated to 90 °C for 3 min. After dilution with water (1.4 mL), the crude product was purified using semi-preparative HPLC (product peak after 9.1min) The collected product was diluted with water (10 mL), trapped on a C18 cartridge (Waters, preconditioned with 5 mL EtOH and 10 mL water), washed with water (5 mL) and eluted with EtOH (0.5 mL). For formulation of the final product **[¹¹C]RS-016,** water for injection (9.5 mL) was added to give an ethanol concentration of 5%. For quality control, an aliquot of the formulated solution was injected into an analytical HPLC system. The identity of the ¹¹C-labeled product was confirmed by comparison with the retention time of its nonradioactive reference compound **RS-016** (10.64 min) and by co-injection. Specific activity of [¹¹C]RS-016 was calculated by comparison of UV peak intensity with a calibration curve of the cold reference compound. **[¹¹C]RS-016** was successfully obtained in ≥99% radiochemical and ≥99% UV purity. The specific activity was 545 ± 154 GBq/µmol with a total activity of 4.42 ± 1.05 GBq at the end of synthesis (n = 39). The total synthesis time from end of bombardment was approximately 35 min.

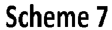

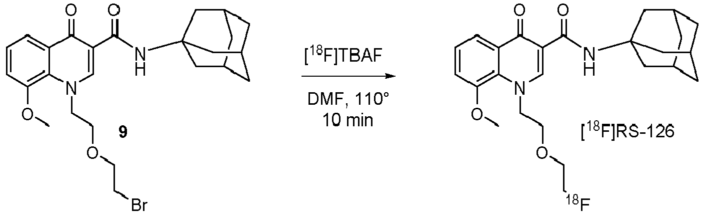

Fluorine-18 nuclide was produced via the ¹⁸O(p,n)¹⁸F nuclear reaction by bombardement of a ¹⁸O-enriched water using a Cyclone 18/9 cyclotron (18-MeV; IBA, Belgium). Aqueous ¹⁸F⁻ was trapped on a hydrophilic anion exchange cartridge (Waters SepPak Accell QMA cartridge carbonate) and eluted with a tetrabutylammonium hydroxide solution (0.2M in MeOH, 1 mL) in a reaction vessel. The elution mixture was azeotropically dried using acetonitrile (3 x 1 mL) at 90 °C under reduced pressure under a gentle flow of nitrogen. A solution of **9** (1 mg) in DMF (0.3 mL) was added and the reaction mixture was stirred for 10 min at 110°. After dilution with water (2.7 mL), the crude product was purified over an ACE C18-300 column (ACE-221-2510) with an isocratic solvent system 0.1% H₃PO₄ in H₂O (40%) and MeCN (60%) at a flow rate of 4 mL/min. The product was diluted with water (10 mL), trapped on a C18 cartridge (Waters, preconditioned with 5 mL EtOH and 10 mL water), washed with water (5 mL) and eluted with EtOH (0.5 mL) through a sterile filter (0.2 µm). EtOH was removed under reduced pressure and the product dissolved in 5% EtOH aqueous solution (2 mL). For quality control, an aliquot of the formulated solution was injected into an analytical Agilent 1100 series HPLC system, equipped with UV multi-wavelength detector and a GabiStar radiodetector (Raytest). An ACE C18-AR column (3 µm, ACE-119-0546) was used with the following conditions: 0.1% TFA in H₂O (solvent A), MeCN (solvent B); 0.0-3.0 min, 30% B; 3.1-13.0 min, 30-95% B; 13.1-15 min, 95% B; flow rate: 1 mL/min. The identity of the ¹⁸F-labeled product was confirmed by comparison with the HPLC retention time of its nonradioactive reference compound **RS-126** and by coinjection. Specific activity of the radiolabeled product was calculated by comparison of UV peak intensity with a calibration curve of the cold reference compound. The specific activity was up to 350 GBq/µmol with a total activity of up to 2.2 GBq at the end of synthesis.

### Example 4- Determination of distribution coefficients (logD)

The partition coefficient *D* was determined by the shake-flask method. Octanol saturated with phosphate buffer pH 7.4 (0.5 mL) and phosphate buffer saturated with octanol (0.5 mL) were mixed with radiotracer of interest (∼3 MBq). The samples were shaken for 15 min and then centrifuged at 5000g for 5 min. Radioactivity in each phase was measured in a gamma counter (Wizard, PerkinElmer). LogD is expressed as the logarithm of the ratio between the radioactivity concentrations (Bq/mL) of the octanol and the buffer phase.

For radiotracers [¹¹C]RS-016 and [¹⁸F]RS-126, a log*D*_{pH 7.4} values of 2.78 and 1.99, respectively, were found.

### Example 5 - In vitro autoradiography of rat and mouse spleen slices

Rodent (mouse and rat) spleen tissue were embedded in TissueTek and cut into 20 µm-thick sections on a Cryostat HM 505 N (Microm) at -15 °C (blade and block). The slices were absorbed on SuperFrost Plus slides (Menzel) and stored at -80° until used. For the experiment, the slices were thawed on ice for 10 min before conditioning in incubation buffer (50 mM TRIS/HCl, 5% BSA, pH 7.4) on ice for 10 min. The slices were then dripped with 600 µL of radioligand solution (0.2 nM) in incubation buffer and incubated for 15 min at rt in a humid chamber. For blockade conditions, the slices were dripped with 600 µL of a mixture of radioligand and GW405833 (5 µM), a specific CB2 partial agonist. After incubation, the slices were washed with washing buffer (50 mM TRIS/HCl, 1% BSA, 5% EtOH, pH 7.4) for 2 min (2 x) and with distilled water for 5 s (2 x) on ice. After drying for 10 min at rt, the slices were exposed (30 min) to appropriate phosphor imager plates (Fuji) and the films were scanned in a BAS5000 reader (Fuji).

Both radiotraces [¹¹C]RS-016 and [¹⁸F]RS-126 performed very well with high binding to spleen, which was displaced by excess of GW405833. Both tracers showed only little unspecific binding, [¹⁸F]RS-126 even less than [¹¹C]RS-016, which is consistent with its lower lipophilicity and similar binding affinity towards CB2.

### Example 6 - Binding specificity / blocking studies

For post mortem biodistribution studies in male Wistar rats, 5-10 MBq of [¹¹C]RS-016 was administered iv via tail vein injection into Wistar rats (n = 3). For blocking conditions, GW405833 (1.5 mg/kg) was injected 30 min before the experiment (n = 3). Animals were sacrificed under anesthesia with isoflurane by decapitation at 15 min post injection (p.i). Organs were collected, weighed and radioactivity measured in a gamma-counter. The accumulated radioactivity in the organs was expressed as part per thousand normalized injected dose per gram of tissue (‰ normalized ID/g tissue).

The highest concentrations of [¹¹C]RS-016 were found in small intestine, liver and spleen, followed by adrenal gland, kidney and pancreas. Concentrations in brain tissue were very low as expected from low CB2 expression levels under healthy conditions. Of the total activity in spleen tissue, 78% was due to specific CB2 binding based on the results under baseline and blocking conditions with 1.5 mg/kg GW405833, demonstrating high specific binding of the tracer towards CB2 *in vivo* in spleen tissue.

| tissue | baseline [‰ID/g] | blocked [‰ID/g] |
|---|---|---|
| spleen | 2.84 ± 0.29 | 0.62 ± 0.11 |
| liver | 5.05 ± 0.33 | 3.80 ± 0.58 |
| kidney | 1.22 ± 0.10 | 1.34 ± 0.26 |
| adrenal gland | 2.16 ± 0.13 | 2.62 ± 0.17 |
| lung | 0.88 ± 0.07 | 0.75 ± 0.11 |
| bone | 0.46 ± 0.04 | 0.39 ± 0.03 |
| heart | 0.80 ± 0.04 | 0.91 ± 0.14 |
| fat | 0.50 ± 0.14 | 0.53 ± 0.13 |
| small intestine | 9.24 ± 2.60 | 4.25 ± 2.95 |
| testis | 0.33 ± 0.05 | 0.34 ± 0.03 |
| blood | 0.44 ± 0.04 | 0.30 ± 0.07 |
| thyroid gland | 0.77 ± 0.07 | 0.64 ± 0.16 |
| urin | 0.60 ± 0.28 | 0.80 ± 0.15 |
| muscle | 0.65 ± 0.05 | 0.71 ± 0.03 |
| pancreas | 1.10 ± 0.05 | 1.13 ± 0.05 |
| skin | 0.50 ± 0.08 | 0.57 ± 0.10 |
| brain | 0.25 ± 0.04 | 0.28 ± 0.01 |

### Example 7 - LPS mouse model of neuroinflammation

As a model of neuroinflammation, six CD1 male mice were injected ip (100-150 µl) with 10 mg/kg lipopolysaccharide (LPS), Escherichia coli strain O111:B4, or vehicle (saline) 5 days prior to PET. For small animal PET brain scans, animals were anesthetized with isoflurane and 10-18 MBq [¹¹C]RS-016 were injected via the tail vein. For blocking conditions, 2.0 mg/kg GW405833 was injected sc 30 min before tracer application. Depth of anesthesia was monitored by measuring respiratory frequency (SA Instruments, Inc., Stony Brook, USA). Body temperature was controlled by a rectal probe and kept at 37 °C by a thermocoupler and a heated air stream. Data were reconstructed in user-defined time frames with a voxel size of 0.3875 x 0.3875 x 0.775 mm³ by 2-dimensional-ordered subsets expectation maximization (2D-OSEM). Random and single but no attenuation correction was applied. PET acquisitions were followed by a CT for anatomical orientation. Image files were analyzed with PMOD 3.5 software (PMOD Technologies Ltd., Zurich, Switzerland). Tissue radioactivity was expressed as standardized uptake values (SUV), that is, the decay-corrected radioactivity per cm³ divided by the injected radioactivity dose per gram of body weight.

The upregulated CB2 gene expression 5 days after 10 mg/kg LPS application was verified by in vitro autoradiography and in vivo PET using [¹¹C]RS-016. Time activity curves (TACs) of mouse whole brain, cortex, hippocampus and cerebellum are shown in Figure 1. Increased [¹¹C]RS-016 accumulation was found for all brain regions after LPS treatment compared to vehicle group (0 mg/kg LPS). This accumulation was reduced in all brain regions after blockade with 2 mg/kg GW405833 to levels of the vehicle group.

### Example 8 - Autoradiography study with post mortem spinal cord slices of ALS patients

To evaluate the potential of our novel CB2 PET tracer in imaging ALS, post mortem spinal cord tissues from ALS patients were investigated in autoradiography experiments. Human post mortem ALS spinal cord tissue samples were embedded in TissueTek and cut into 20 µm-thick sections on a Cryostat. Autoradiography was performed as described in section "Example 5". Samples were incubated with 0.2 nM [¹¹C]RS-016 in the absence or presence of 5 µM GW405833 as blocking agent.These experiments displayed high specific binding of [¹¹C]RS-016 to diseased tissue.

## Claims

1. Compounds according to formula (I) or (II): 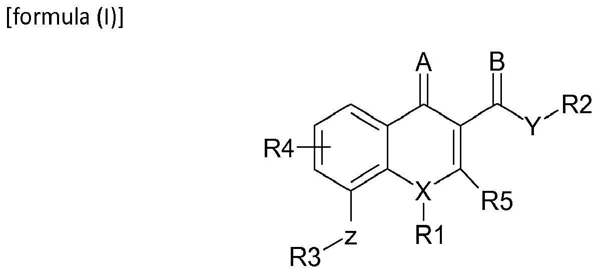 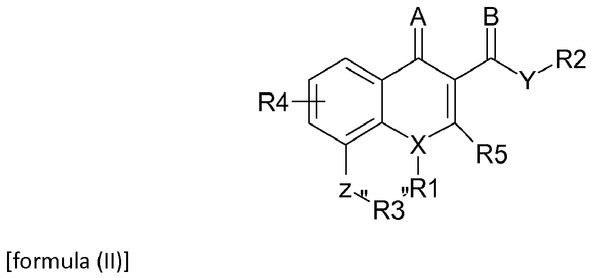 wherein:
A is selected from -O-, -S- and -NR₆-, preferably A is -O-;
B is selected from -O-, -S-, -NR₆-, preferably B is -O-;
X is -N- or -CH-, preferably -N-;
Y is selected from -O-, -NH-, -NR₆-, -S-, substituted or non-substituted -CH₂- or a direct bond, preferably Y is -NH-;
Z is selected from -O-, -NH-, -S-, substituted or non-substituted -CH₂- or a direct bond, preferably Z is -O-;
R₁ is selected from the group consisting of
(i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
(ii) linear or branched, substituted or non-substituted (C₁₋₁₀)alkyl thioether, (C₂₋₁₀)-alkenyl thioether, (C₂₋₁₀)alkynyl thioether, (C₄₋₁₀)carbocyclic thioether;
(iii) linear or branched, substituted or non-substituted (C₂₋₁₀) NHR₆ or N(R₆)₂, wherein one or both R₆ are independently selected or together form a substituted or non-substituted (C₃₋₁₀)carbocyclic amine;
(iv) linear or branched, substituted or non-substituted (C₂₋₁₀)alkoxyalkyl, preferably 2-ethoxyethyl, 2-fluorethoxyethyl;
R2 is substituted or non-substituted (3s, 5s, 7s)adamantyl, preferably substituted or non-substituted (3s, 5s, 7s)adamant-1-yl, more preferably 3-substituted (3s, 5s, 7s)adamant-1-yl, wherein the 3-substitutent is preferably selected from the group consisting of hydroxy, amino, -NHR₆, -NH(R₆)₂, wherein each R₆ is selected independently from one another, thio, (C₁₋₁₀)alkyl, (C₂₋₁₀)alkenyl, (C₁₋₁₀)alkinyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, halogen, preferably Cl or F, most preferably R2 is 3-hydroxy- or 3-fluoro(3s, 5s, 7s)adamant-1-yl;
R3 is linear or branched, substituted or non-substituted (C₁₋₁₀)alkyl, (C₂₋₁₀)alkenyl, (C₂₋₁₀)-alkynyl, (C₃₋₁₀)carbocycle, preferably (C₃₋₆)cylcoalkyl or a (C₅₋₆)heterocycle having 1 or 2 heteroatoms each independently selected from N, O or S, preferably R3 is (C₁₋₅)alkyl, most preferably methyl, ethyl, propyl;
R4 is H, F, Cl, Br, -CF₃, -CF₂CH₃, cyano, nitro, linear or branched, substituted or non-substituted (C₁₋₁₀)alkyl, (C₂₋₁₀)alkenyl, (C₂₋₁₀)alkynyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, preferably R4 is H;
R5 is H, F, Cl, Br, -CF₃, -CF₂CH₃, cyano, nitro, linear or branched, substituted or non-substituted (C₁₋₁₀)alkyl, (C₂₋₁₀)alkenyl, (C₂₋₁₀)alkynyl, (C₁₋₁₀)alkoxy, (C₃₋₁₀)carbocycle, preferably (C₃₋₆)cylcoalkyl or a (C₅₋₆)heterocycle having 1 or 2 heteroatoms each independently selected from N, O or S, preferably R5 is H;
R₆ is linear or branched, substituted or non-substituted (C₁₋₁₀)alkyl, (C₂₋₁₀)alkenyl, (C₂₋₁₀)-alkynyl, (C₃₋₁₀)carbocycle, preferably (C₃₋₆)cylcoalkyl or a (C₅₋₆)heterocycle having 1 or 2 heteroatoms each independently selected from N, O or S;
or R3 and R₁ together form a substituted or non-substituted 6-membered heterocyclic ring, wherein X is N or CH, Z is selected from -O-, -NH-, -NR₆-, -S-, substituted or non-substituted -CH₂-, R₁ and R3 are independently of one another substituted or non-substituted -CH₂-, with the proviso that at least one of R₁ or R3 is substituted by
(i) linear or branched, substituted or non-substituted (C₁₋₁₀)alkyl ether, (C₂₋₁₀)alkenyl ether, (C₂₋₁₀)alkynyl ether, (C₄₋₁₀)carbocyclic ether;
(ii) linear or branched, substituted or non-substituted (C₁₋₁₀)alkyl thioether, (C₂₋₁₀)-alkenyl thioether, (C₂₋₁₀)alkynyl thioether, (C₄₋₁₀)carbocyclic thioether;
(iii) linear or branched, substituted or non-substituted (C₂₋₁₀) NHR₆ or N(R₆)₂, wherein one or both R₆ are independently selected or together form a substituted or non-substituted (C₃₋₁₀)carbocyclic amine;
(iv) linear or branched, substituted or non-substituted (C₂₋₁₀)alkoxyalkyl, preferably 2-ethoxyethyl, 2-fluorethoxyethyl;
(v) hydroxy, thiol, amino,
and pharmaceutically acceptable salts or solvates thereof.

2. Compound according to claim 1, wherein
(i) A and B are both -O-; and/or
(ii) X is N and Y is -NH-; and/or
(iii) Z is -O-; and/or
(iv) R4 or R5, preferably both R4 and R5 are H.

3. Compound according to claims 1 or 2, wherein R₁ is selected from the group consisting of
(i) linear or branched, substituted or non-substituted (C₁₋₈)alkyl-, alkenyl-, alkynyl ether, (C₄₋₈)carbocyclic ether, (C₁₋₄)alkyl-, alkenyl-, alkynyl ether, (C₄₋₅)carbocyclic ether;
(iii) linear or branched, substituted or non-substituted (C₁₋₈) NHR₆ or N(R₆)₂, wherein one or both R₆ are independently selected or together form a substituted or non-substituted (C₄₋₈)carbocyclic amine;
(iv) linear or branched, substituted or non-substituted (C₂₋₈)alkoxyalkyl, preferably (C₂₋₆)alkoxyalkyl, more preferably (C₂₋₄)alkoxyalkyl, most preferably 2-ethoxyethyl, 2-fluorethoxyethyl and chloroethoxyethyl.

4. Compound according to any of claims 1 to 3, wherein R2 is 3-substituted (3s, 5s, 7s)-adamant-1-yl, wherein the 3-substitutent is preferably selected from the group consisting of hydroxy, amino, -NHR₆, -NH(R₆)₂, wherein each R₆ is selected independently from one another, hydroxythio, (C₁₋₈)- alkyl, alkenyl-, alkinyl-, alkoxy, preferably (C₁₋₄)- alkyl, alkenyl-, alkinyl-, alkoxy, (C₃₋₆)cylcoalkyl, (C₅₋₆)heterocycle having 1 or 2 heteroatoms each independently selected from N, O or S, halogen, preferably Cl or F, most preferably R2 is 3-hydroxy- or 3-fluoro(3s, 5s, 7s)adamant-1-yl;

5. Compound according to any of claims 1 to 4, wherein R3 is linear or branched, substituted or non-substituted (C₁₋₈)-alkyl, alkenyl, alkynyl, (C₃₋₈)carbocycle, preferably (C₃₋₆)cylcoalkyl or a (C₅₋₆)heterocycle having 1 or 2 heteroatoms each independently selected from N, O or S, more preferably R3 is substituted or unsubstituted (C₁₋₅)alkyl, most preferably methyl, ethyl, propyl;

6. Compound according to any of claims 1 to 5, wherein
(i) R4 is H, F, Cl, Br, -CF₃, -CF₂CH₃, cyano, nitro, linear or branched, substituted or non-substituted (C₁₋₄)alkyl, (C₂₋₄)alkenyl, (C₂₋₄)alkynyl, (C₁₋₄)alkoxy, (C₃₋₆)carbocycle, preferably (C₃₋₆)cylcoalkyl or a (C₅₋₆)heterocycle having 1 or 2 heteroatoms each independently selected from N, O or S, most preferably R4 is H; and/or
(ii) R5 is H, F, Cl, Br, -CF₃, -CF₂CH₃, cyano, nitro, linear or branched, substituted or non-substituted (C₁₋₄)alkyl, (C₂₋₄)alkenyl, (C₂₋₄)alkynyl, (C₁₋₄)alkoxy, (C₃₋₆)carbocycle, preferably (C₃₋₆)cylcoalkyl or a (C₅₋₆)heterocycle having 1 or 2 heteroatoms each independently selected from N, O or S, most preferably R5 is H.

7. Compound according to any of claims 1 to 6, wherein R₆ is linear or branched, substituted or non-substituted (C₁₋₆)alkyl, alkenyl, alkynyl, preferably (C₁₋₄)alkyl, alkenyl, alkynyl, (C₃₋₆)carbocycle, preferably (C₃₋₆)cylcoalkyl or a (C₅₋₆)heterocycle having 1 or 2 heteroatoms each independently selected from N, O or S;

8. Compound according to any of claims 1 to 7 and formula (II), wherein R3 and R1 together form a substituted or unsubstituted 6-membered heterocyclic ring, wherein X is N or CH, Z is selected from -O-, -NH-, -NR₆-, -S-, substituted or unsubstituted -CH₂-, R₁ and R3 are independently of one another substituted or unsubstituted -CH₂-, with the proviso that at least one of R₁ or R3 is substituted by
(i) linear or branched, substituted or non-substituted (C₁₋₈)alkyl ether, (C₂₋₈)alkenyl ether, (C₂₋₈)alkynyl ether, (C₄₋₈)carbocyclic ether;
(ii) linear or branched, substituted or non-substituted (C₁₋₈)alkyl thioether, (C₂₋₈)alkenyl thioether, (C₂₋₈)alkynyl thioether, (C₄₋₈)carbocyclic thioether;
(iii) linear or branched, substituted or non-substituted (C₂₋₈) NHR₆ or N(R₆)₂, wherein one or both R₆ are independently selected or together form a substituted or non-substituted (C₃₋₈)carbocyclic amine;
(iv) linear or branched, substituted or non-substituted (C₂₋₈)alkoxyalkyl, preferably (C₂₋₄)alkoxyalkyl, more preferably 2-ethoxyethyl or 2-fluorethoxyethyl;
(v) hydroxy, thiol, amino.

9. Compound according to any of claims 1 to 8, wherein
A and B are -O-, X is N or -CH-, Y is -O- or -NH-, Z is -O-, R4 and R5 are both H,
R₁ is -(C(R7)₂)ₙ-M-C(R8)₂)ₘ-C(R9)₃, wherein n is 1 to 4 and m is 0 to 4, M is -O-, NH, NR₆, or -S-, and each of R7, R8 and R9 is independently selected from H and halogen, preferably H, Cl and F;
R2 is 3-substituted (3s, 5s, 7s)adamant-1-yl, wherein the 3-substitutent is preferably selected from the group consisting of hydroxy, amino, -NHR₆, -NH(R₆)₂, wherein each R₆ is selected independently from one another, thio, (C₁₋₄)-alkyl, halogen, preferably Cl or F;
R3 is linear or branched, substituted or non-substituted (C₁₋₅)alkyl, (C₂₋₅)alkenyl, (C₂₋₅)-alkynyl, (C₃₋₆)carbocycle, preferably (C₃₋₆)cylcoalkyl or a (C₅₋₆) heterocycle having 1 or 2 heteroatoms each independently selected from N, O or S, preferably R3 is (C₁₋₅)alkyl, most preferably methyl, ethyl, propyl.

10. Compound according to any of claims 1 to 9, wherein the compound is selected from the group consisting of
(i) *N*-(1-adamantyl)-1-butyl-8-methoxy-4-oxo-1,4-dihydroquinoline-3-carboxamide
(ii) *N*-(*tert*-butyl)-1-butyl-8-methoxy-4-oxo-1,4-dihydroquinoline-3-carboxamide
(iii) 1-butyl-*N*-cyclopentyl-8-methoxy-4-oxo-1,4-dihydroquinoline-3-carboxamide
(iv) 1-butyl-*N*-(cyclopropylmethyl)-8-methoxy-4-oxo-1,4-dihydroquinoline-3-carboxamide
(v) *N*-(*tert*-butyl)-1-(3-fluoropropyl)-8-methoxy-4-oxo-1,4-dihydroquinoline-3-carboxamide
(vi) *N*-(1-adamantyl)-1-(2-ethoxyethyl)-8-methoxy-4-oxo-1,4-dihydroquinoline-3-carboxamide
(vii) 1-(2-ethoxyethyl)-8-methoxy-4-oxo-*N*-phenethyl-1,4-dihydroquinoline-3-carboxamide
(viii) 1-(2-ethoxyethyl)-*N*-(3-hydroxyadamantan-1-yl)-8-methoxy-4-oxo-1,4-dihydroquinoline-3-carboxamide
(ix) 1-(2-ethoxyethyl)-*N*-(3-fluoroadamantyl)-8-methoxy-4-oxo-1,4-dihydroquinoline-3-carboxamide
(x) *N*-(1-adamantanyl)-1-(2-ethoxyethyl)-8-(2-fluoroethoxy)-4-oxo-1,4-dihydroquinoline-3-carboxamide
(xi) *N*-(1-adamantyl)-1-(2-(2-fluoroethoxy)ethyl)-8-methoxy-4-oxo-1,4-dihydroquinoline-3-carboxamide

11. Compound according to any of claims 1 to 10, wherein the compound has a distribution coefficient (logD) in 1-octanol, phosphate buffer at pH 7.4 of at most 3.5, preferably at most 3.0, more preferably at most 2.8, most preferably at most 2.

12. Compound according to any of claims 1 to 11, wherein the compound is radiolabeled, preferably radiolabeled in any of R₁, R₂ or R₃, preferably in R₃ or R₂, most preferably in R_{3;} preferably radiolabelled by an isotope selected from the group consisting of non-metallic position emitting isotopes and ¹¹C, ¹⁸F.

13. Use of a compound according to any of claims 1 to 12
(i) for determining cannabinoid receptor 2 (CB2)-selective receptor localization and/or density, preferably in the central nervous system (CNS), the peripheral nervous system (PNS), heart, liver, gastrointestinal tract, spleen, pancreas, kidney, testis, ovary and/or the prostate; or
(ii) for determining cannabinoid receptor 2 (CB2)-upregulation in microglia.

14. Compound according to any of claims 1 to 12 for use in the diagnosis, prophylaxis and/or therapy of
(i) CB2 receptor-related diseases, preferably selected from the group of CB2 receptor-related diseases consisting of cardiovascular disease, myocardial infarction, ischemia reperfusion injury, heart failure, cardiomyopathies, atherosclerosis, restenosis, stroke, spinal cord injury, cirrhotic cardiomyopathy, septic shock by live bacteria, hepatic ischaemia reperfusion injury, obesity, non-alcoholic fatty liver disease, diabetes, diabetic complications, obesity/metabolic syndrome; liver, gastrointestinal and skin diseases; liver fibrosis, cirrhosis, alcohol-induced liver injury, pancreatitis, inflammatory bowel disease, colitis, diverticulitis, nephropathy, neurodegenerative/neuroinflammatory disorders, in particular multiple sclerosis (MS), Alzheimer's disease (AD), Parkinson's disease (PD) and Huntington's disease (HD); spinal cord injury, pain; psychiatric disorders, in particular anxiety and depression schizophrenia; rheumatoid arthritis, cachexia, cancer, chemotherapy-induced nausea and vomiting; or
(ii) neuroinflammatory and neurodegenerative diseases, preferably diseases selected from the group consisting of multiple sclerosis (MS), amyotrophic lateral sclerosis (ALS), Alzheimer's disease (AD) and Parkinson's disease (PD).

15. Compound according to any of claims 1 to 12 for use in the prophylaxis and/or therapy of pain, preferably hyperalgesia.
